# EUROPEAN PATENT SPECIFICATION

(11) **EP 4 114 420 B1**
(45) Date of publication and mention of the grant of the patent: **03.06.2026**
(21) Application number: 21709003.4
(22) Date of filing: 04.03.2021
(51) Int. Cl.: A61K 35/74, A61P 43/00

(54) **COPROCOCCUS BACTERIA FOR USE IN THE TREATMENT OF METABOLIC SYNDROME AND INFLAMMATORY BOWEL DISEASES**
COPROCOCCUS-BAKTERIEN ZUR VERWENDUNG IN DER BEHANDLUNG VON METABOLISCHEM SYNDROM UND ENTZÜNDLICHEN DARMERKRANKUNGEN
BACTÉRIES COPROCOCCUS POUR L'UTILISATION DANS LE TRAITEMENT DU SYNDROME MÉTABOLIQUE ET DE MALADIES INTESTINALES INFLAMMATOIRES

(30) Priority: 04.03.2020 EP 20305228
(43) Date of publication of application: 11.01.2023
(73) Proprietor: Institut national de recherche pour l'agriculture, l'alimentation et l'environnement, 75007 Paris (FR); Assistance Publique Hôpitaux de Paris, 75004 Paris (FR); SORBONNE UNIVERSITE, 75006 Paris (FR); Institut National de la Santé et de la Recherche Médicale (INSERM), 75013 Paris (FR)
(72) Inventor: SOKOL, Harry, 75015 PARIS (FR); AGUS, Allison, 91300 MASSY (FR); MICHAUDEL, Chloé, 78150 LE CHESNAY (FR); PLANCHAIS, Julien, 78220 VIROFLAY (FR); LANGELLA, Philippe, 78140 VELIZY (FR)
(74) Representative: Cabinet Becker et Associés
(86) International application number: PCT/EP2021/055478
(87) International publication number: WO 2021/175998

(56) References cited:
- WO-A2-2014/145958
- W J SANDBORN ET AL: "UEG Week 2018 Oral Presentations OP242", UNITED EUROPEAN GASTROENTEROLOGY JOURNAL : UEG JOURNAL, vol. 6, no. 8_suppl, 1 October 2018 (2018-10-01), GB, pages A1 - A134, XP055670712, ISSN: 2050-6406, DOI: 10.1177/2050640618792817

## Description

### FIELD OF THE INVENTION

The present invention relates to the field of medicine and in particular to compositions comprising bacterial probiotics for use in the preventive or curative treatment of metabolic syndrome and associated disorders, and inflammatory bowel diseases.

### BACKGROUND OF THE INVENTION

The metabolic syndrome is a cluster of the most dangerous heart attack and diabetes risk factors. This represents a major health problem because a quarter of the world's adults have metabolic syndrome. People with metabolic syndrome are twice as likely to die from, and three times as likely to have a heart attack or stroke compared with people without the syndrome. People with metabolic syndrome have a five-fold greater risk of developing type 2 diabetes.

Inflammatory bowel diseases (IBD) is a general term used to identify a group of inflammatory disorders of the gastrointestinal (GI) tract including Crohn's disease and ulcerative colitis. While the exact cause of IBD is not entirely understood, it is known to involve genetic predisposition, dysbiosis of the gut microbiota and environmental influences. IBD is characterized by the repeated alternating cycles of clinical relapse and remission. In the absence of adequate treatment, IBD leads to chronic inflammation and thus to irreversible intestinal damages.

Crohn's disease can affect any part of the GI tract but most commonly affects the end of the small intestine (the ileum) where it joins the beginning of the colon. Crohn's disease may appear in "patches," affecting some areas of the GI tract while leaving other sections completely untouched. In Crohn's disease, the inflammation may extend through the entire thickness of the bowel wall. Ulcerative colitis is limited to the large intestine (colon) and the rectum. The inflammation occurs only in the innermost layer of the lining of the intestine. It usually begins in the rectum and lower colon, but may also spread continuously to involve the entire colon. In some individuals, it is difficult to determine whether their IBD is Crohn's disease or ulcerative colitis. In these rare cases, people are given the diagnosis of indeterminate colitis (IC).

International patent application WO 2014/145958 discloses therapeutic compositions containing combinations of bacteria, for the maintenance or restoration of a healthy microbiota in the gastrointestinal tract of a mammalian subject, and methods for treating, preventing, or reducing the severity of a disorder selected from the group consisting of Clostridium difficile associated diarrhea, type 2 diabetes, obesity, irritable bowel disease, colonization with a pathogen or pathobiont, and infection with a drug-resistant pathogen or pathobiont. It was also disclosed that the abundance of butyrate-producing bacteria with antiinflammatory properties *(Subdoligranulum, Eubacterium, Faecalibacterium, Coprococcus, Ruminococcus,* etc.) was significantly decreased in IBD patients comparing to healthy subject (Danilova et al. UEG Week 2018 Oral Presentations OP334, United European Gastroenterology journal, vol. 6, no. 8 suppl, 1 October 2018, pages A1-A134).

Some recent studies have revealed that the metabolic syndrome and IBD can be treated or prevented using aryl hydrocarbon receptor (AhR) agonists or bacterial probiotics producing such agonists (see WO 2018/065132 and WO 2017/032739). Aryl-hydrocarbon receptor (AhR) is a ligand-activated nuclear receptor/transcription factor that regulates genes involved in toxicant metabolism and provides a major defense to environmental exposures. The AhR can be activated by dietary components such as fats and fat derivatives, and there is evidence linking the activated AhR to major diseases, including obesity (La Merill et al, 2009, Environ Health Perspect, 117, 1414-1419).

In a previous patent application (WO 2018/065132), the inventors observed that animal models of metabolic syndrome (high fat diet (HFD)-induced metabolic syndrome or leptin deficient mice (ob/ob mice)) are associated with a decreased AhR agonist activity of their gut microbiota and the administration of AhR agonist, either via a pharmacological strategy or via an intestinal bacterium naturally producing AhR agonist, reduces the weight gain, and improves glucose tolerance, insulin sensitivity and fatty liver disease. They also observed that, in human, the AhR agonist activity of the gut microbiota is inversely correlated with the metabolic syndrome. Furthermore, in another previous patent application (WO 2017/032739), the inventors demonstrated that inoculation with lactobacilli that metabolize tryptophan and produce AHR ligands reduces colitis in an AHR-dependent manner.

However, even if the link between AhR and the metabolic syndrome or IBD has been elucidated, there is still a strong need of bacterial probiotics exhibiting suitable features to be used in the prevention or treatment of these pathologies.

### SUMMARY OF THE INVENTION

The inventors herein identified a bacterial probiotic associated with a high AhR activity. Indeed, they demonstrated that *Coprococcus comes* bacteria and/or a culture supernatant thereof, are able to protect mice from DSS-induced colitis, to alleviate diet-induced metabolic impairments, and to reduce hepatic steatosis features such as cholesterol and HDL serum levels in high-fat diet (HFD) mice. All these effects were shown to be AhR dependent.

The present invention is defined by the appended set of claims.The present invention relates to a composition comprising a *Coprococcus* bacterium and/or a culture extract thereof exhibiting AhR agonist activity, for use in the treatment of a disease selected from the group consisting of metabolic syndrome and associated disorders being selected from the group consisting of cardiovascular diseases, insulin resistance, glucose intolerance, non alcoholic fatty liver disease and lipodystrophy, and inflammatory bowel diseases.

Preferably, the bacterium is a *Coprococcus comes* bacterium.

More preferably, the bacterium is *Coprococcus comes* strain deposited with the Deutsche Sammlung von Mikroorganismen und Zellkulturen (DSMZ) under accession number DSM 33359, or a functionally equivalent mutant thereof.

Preferably, the *Coprococcus* bacterium in the composition is a living *Coprococcus* bacterium and/or the culture extract is a culture supernatant. In particular, the composition may comprise a living *Coprococcus* bacterium and a culture supernatant thereof, preferably a living *Coprococcus comes* bacterium and a culture supernatant thereof.

Optionally, the composition may further comprise one or several additional bacterial probiotics capable of producing an AhR agonist and selected from the group consisting of bacteria belonging to the genera *Allobaculum, Adlercreutzia, Anaerostipes, Bifidobacterium, Propionibacterium, Bacteroides, Eubacterium, Enterococcus, Ruminococcus* and *Faecalibacterium, Escherichia coli,* and lactic acid bacteria such as bacteria belonging to the genera *Lactobacillus* and *Streptococcus,* or selected from the group consisting of bacteria belonging to the genera *Allobaculum, Adlercreutzia, Propionibacterium, Enterococcus, Escherichia coli, Lactobacillus* and *Streptococcus.* In particular, the composition may further comprise one or several additional bacterial probiotics selected from the group consisting of strains available under CNCM deposit numbers CNCM 1-5019, CNCM I-5020, CNCM I-5021, CNCM I-5022 and CNCM I-5023, *Ruminococcus gnavus* ATCC 29149, *Lactobacillus salivarius* DSM 20555, *Lactobacillus reuteri* DSM 20016, *Lactobacillus gasseri* DSM 20243, *Faecalibacterium prausnitzii* A2-165, *Escherichia coli* MG1665, *Anaerostipes hadrus* DSM *3319, Anaerostipes caccae* DSM 14662, *Anaerostipes butyraticus* DSM 22094 and *Allobaculum stercoricanis* DSM 13633. Alternatively, in another embodiment, the composition does not comprise any additional bacterium, in particular any additional bacterial probiotic.

Preferably, the composition is to be administered by oral or rectal route.

Preferably, the disease is selected from metabolic syndrome or associated disorders, said disorders being selected from the group consisting of cardiovascular diseases, insulin resistance, glucose intolerance, non alcoholic fatty liver disease and lipodystrophy. The cardiovascular diseases are preferably coronary heart diseases, more preferably heart attack or stroke.

In embodiments wherein the disease is an inflammatory bowel disease, the disease is preferably selected from the group consisting of Crohn's disease, ulcerative colitis, indeterminate colitis (IC), other noninfective gastroenteritis, enteritis, enterocolitis and colitis, and pouchitis, more preferably is selected from Crohn's disease and ulcerative colitis. Preferably, the disease is not antibiotic associated colitis, more preferably is not *Clostridium difficile* associated diarrhea.

In another aspect, the present invention also relates to a *Coprococcus comes* strain deposited with the Deutsche Sammlung von Mikroorganismen und Zellkulturen (DSMZ) under accession number DSM 33359, or a functionally equivalent mutant thereof.

### BRIEF DESCRIPTION OF THE FIGURES

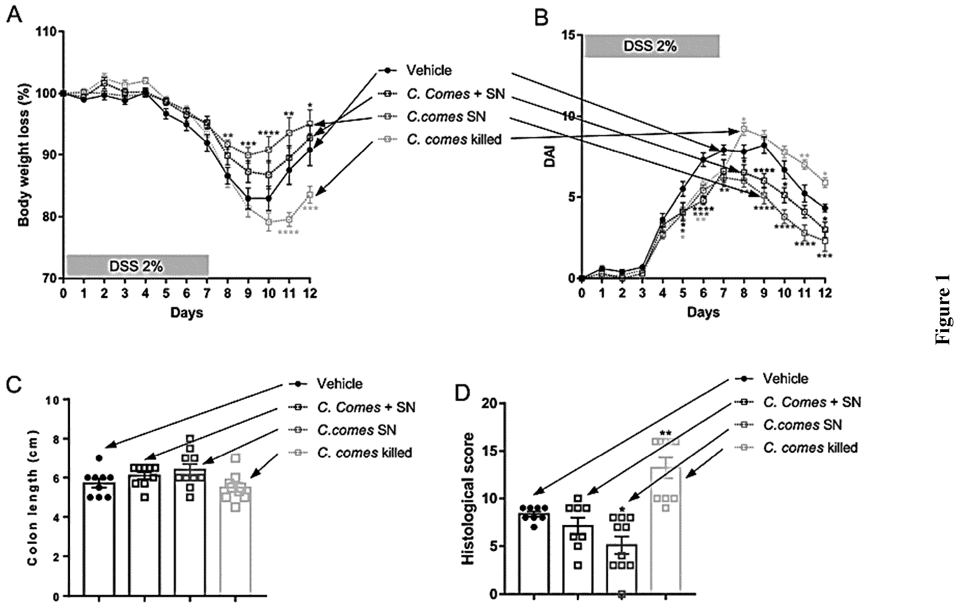

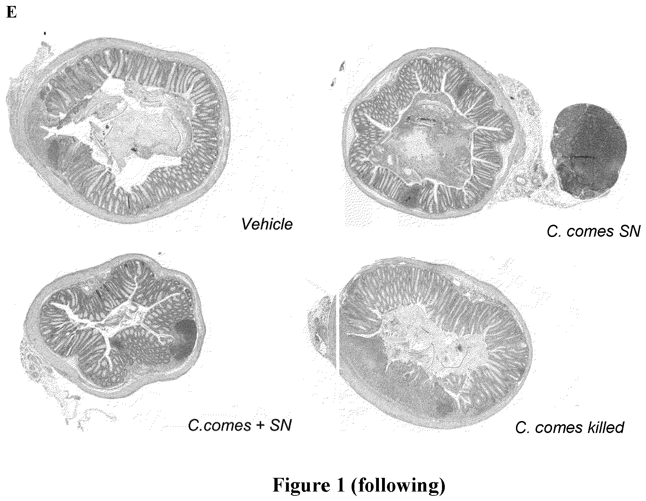

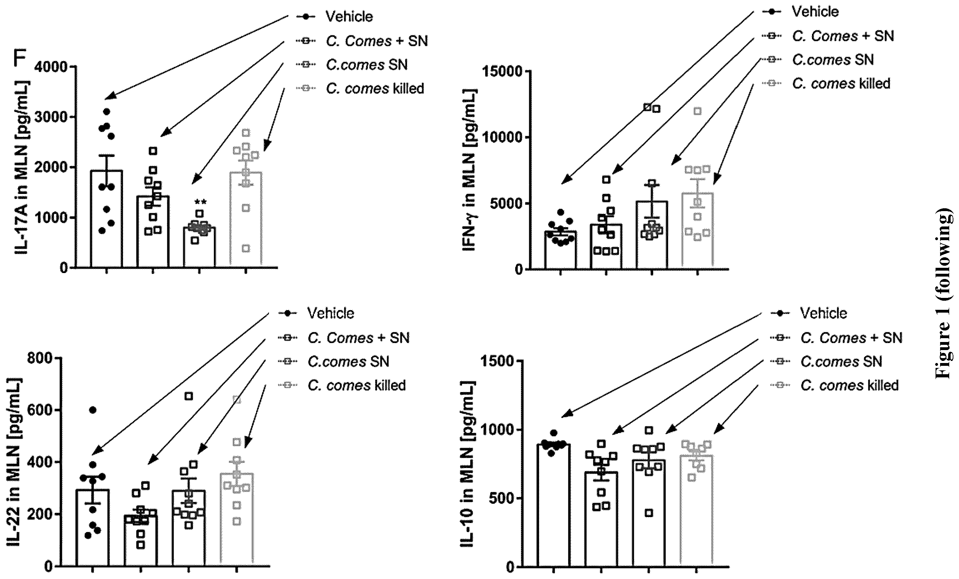

**Figure 1** : *C. comes* supernatant protects from DSS-induced colitis. Body weight loss **(A),** DAI **(B)** during DSS-induced colitis in mice treated with C. comes (bacteria + supernatant, supernatant or killed bacteria). Colon length **(C)** and histological score after HES staining **(D-E),** after 12 days of DSS-induced colitis. IL-17A, IFN-y, IL-22 and IL-10 proteins after MLN cells stimulation at day 12 **(F).** Each graph is representative of two distinct experiments n=5-10. Statistics : Two-way ANOVA, Bonferonni post test.

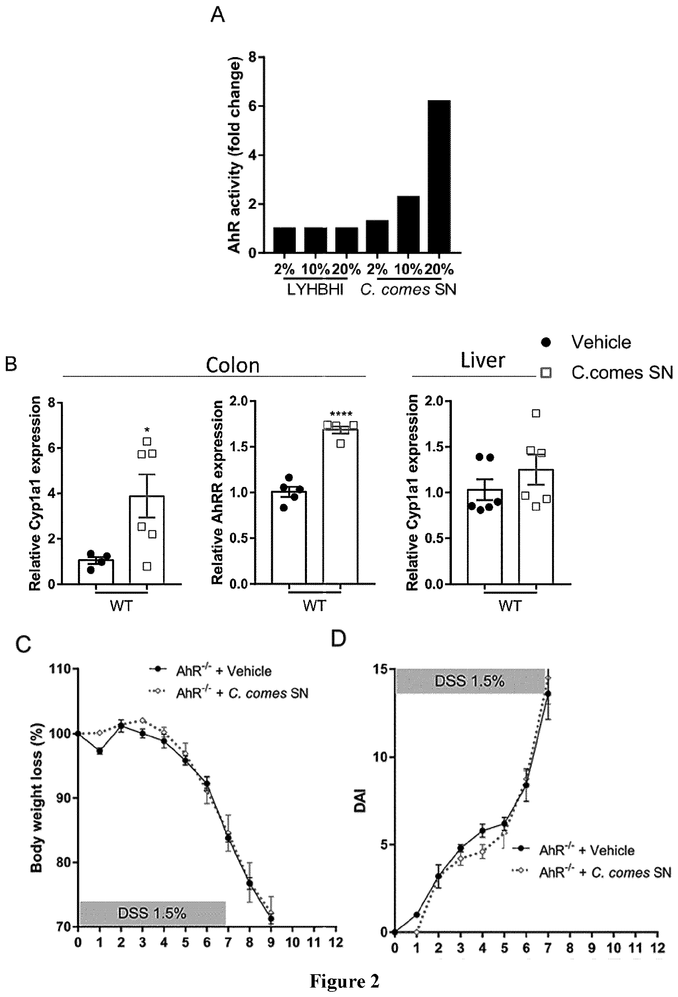

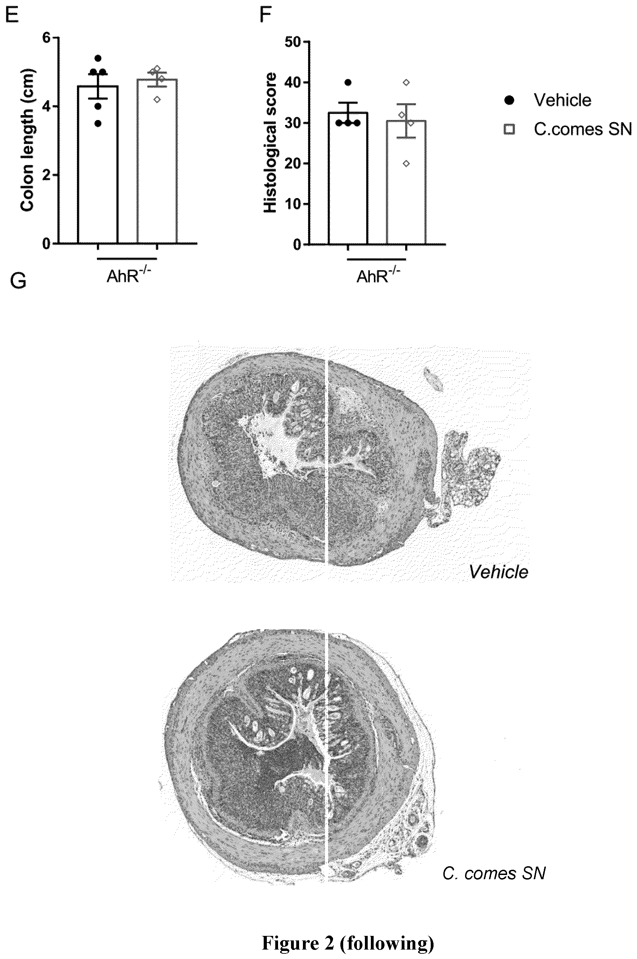

**Figure 2** : *C. comes* protection is AhR dependent. Fold change of AhR activity in H1L1.1c2 **(A),** Cyp1a and AhRR mRNA in colon and liver at steady state after *C*. *comes* supernatant gavage during 3 weeks **(B).** Body weight loss **(C)** and DAI (D) after DSS-induced colitis in AhR -/- with or without *C*. *comes* SN gavage. Colon length **(E),** histological score **(F)** and HES staining after 9 days of colitis **(G).** Each graph is representative of two distinct experiments n=5-10. Statistics : Two-way ANOVA, Bonferonni post test.

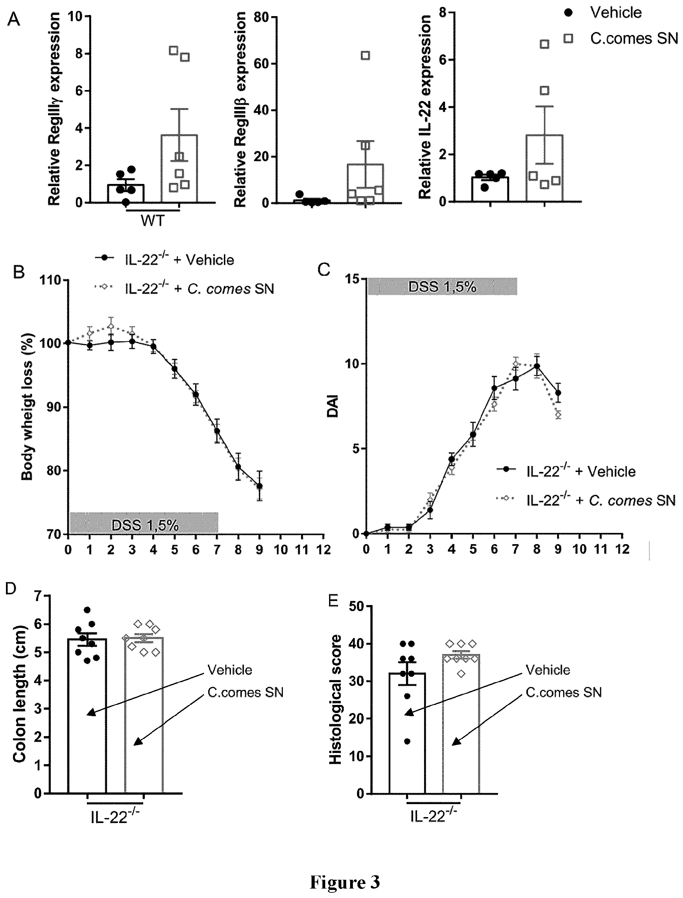

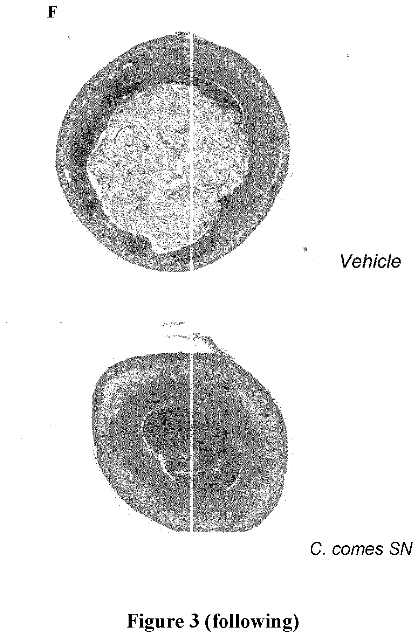

**Figure 3** : *C. comes* protection passes though IL-22 secretion. Fold change of Reg3y, Reg3β and IL-22 mRNA in colon at steady state after C. *comes* supernatant gavage during 3 weeks **(A).** Body weight loss **(B)** and DAI **(C)** after DSS-induced colitis in IL22 -/- mice. Colon length **(D),** histological score **(E)** and HES staining after 9 days of colitis **(F).** Each graph is representative of two distinct experiments n=7-8. Statistics : Two-way ANOVA, Bonferonni post test.

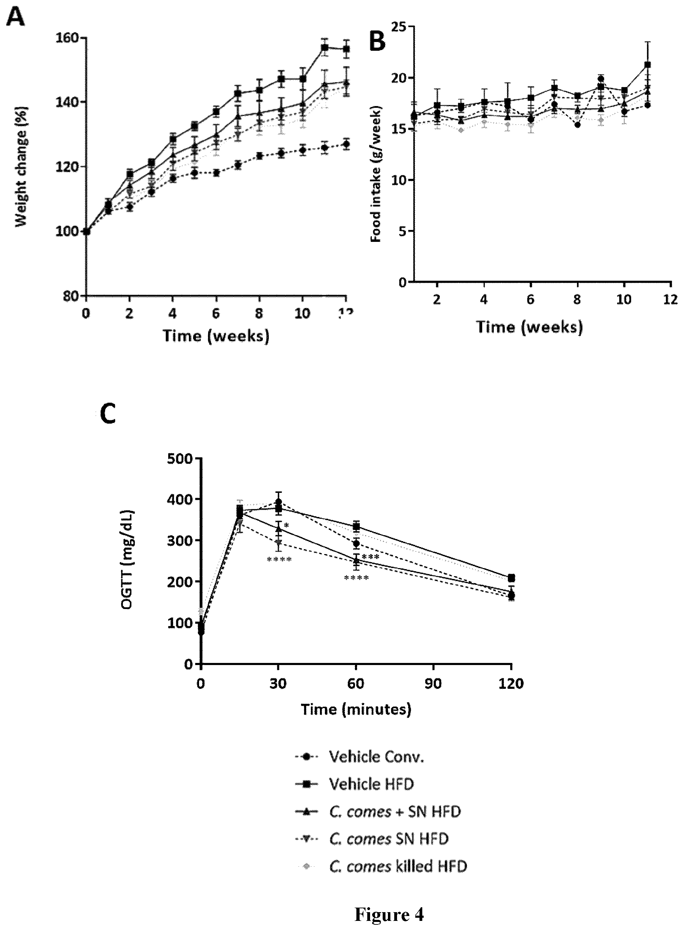

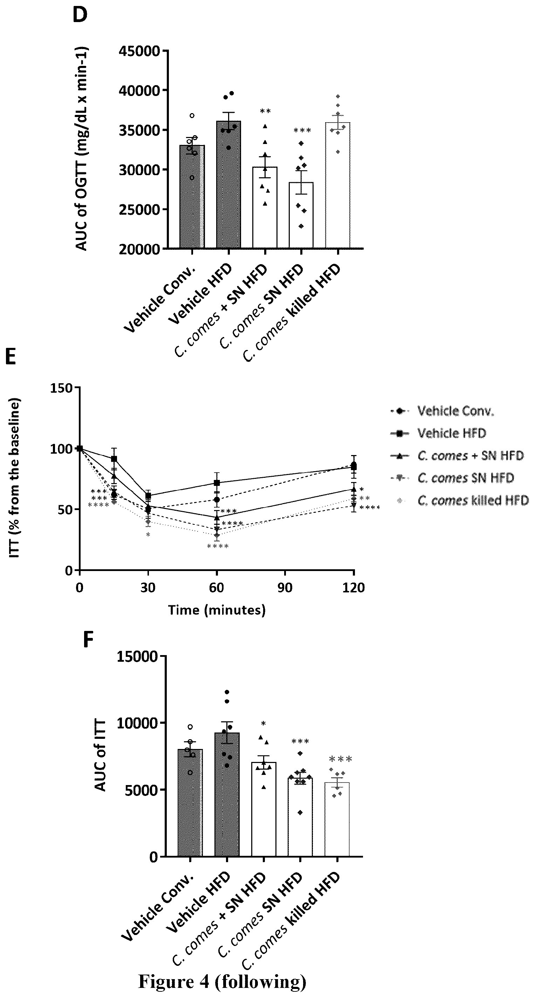

**Figure 4** : Treatment with *Coprococcus comes* alleviates diet-induced metabolic impairments. **(A)** Body weight gain, **(B)** Food intake, **(C)** Blood glucose level after 16h of fasting of Conv- and HFD-fed mice supplemented with C. comes or vehicle. **(D)** AUC (area under the curve) of the OGTT, (E) Blood glucose levels after 4h of fasting during the ITT, **(F)** AUC of the ITT.

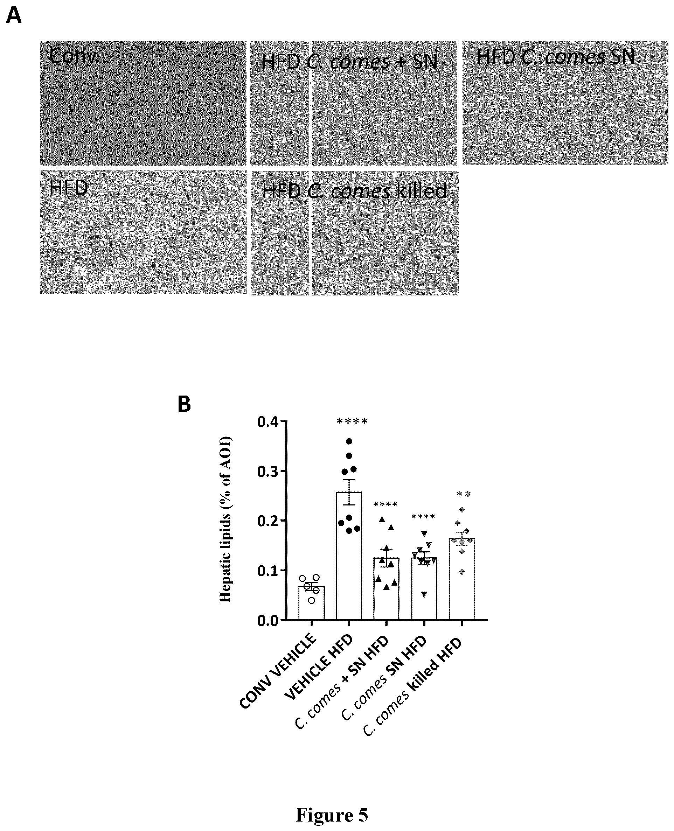

**Figure 5** : Treatment with *Coprococcus comes* reduces hepatic steatosis in HFD mice. **(A)** Representative pictures of H&E-stained liver sections from indicated mice. **(B)** Lipid area, calculated as the percent area of interest (AOI), in the liver cross-sections of Conv- and HFD-fed mice supplemented with C. *comes* or vehicle.

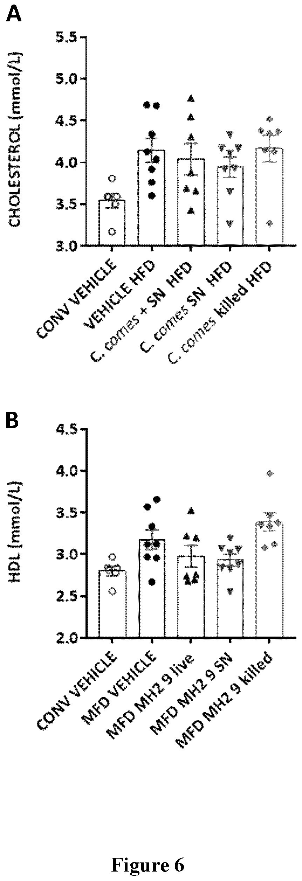

**Figure 6** : Treatment with *Coprococcus comes* reduces serum features of HFD-induced metabolic syndrome. Concentrations of **(A)** cholesterol and **(B)** HDL from the sera of indicated mice.

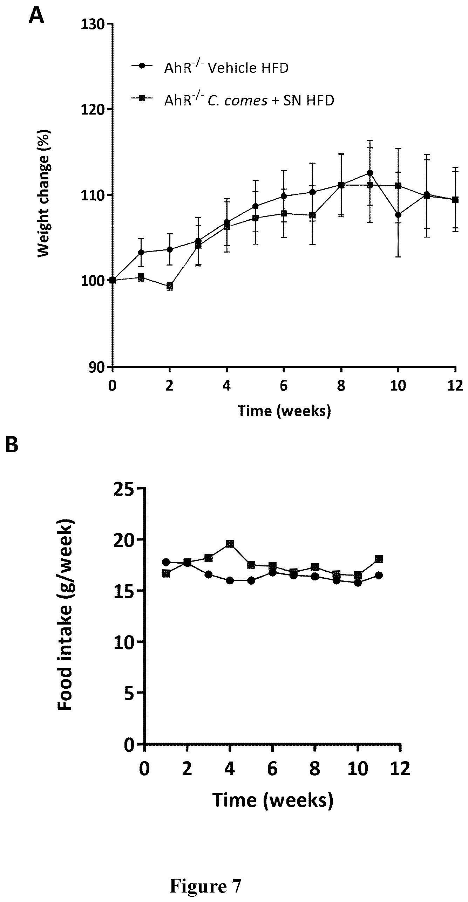

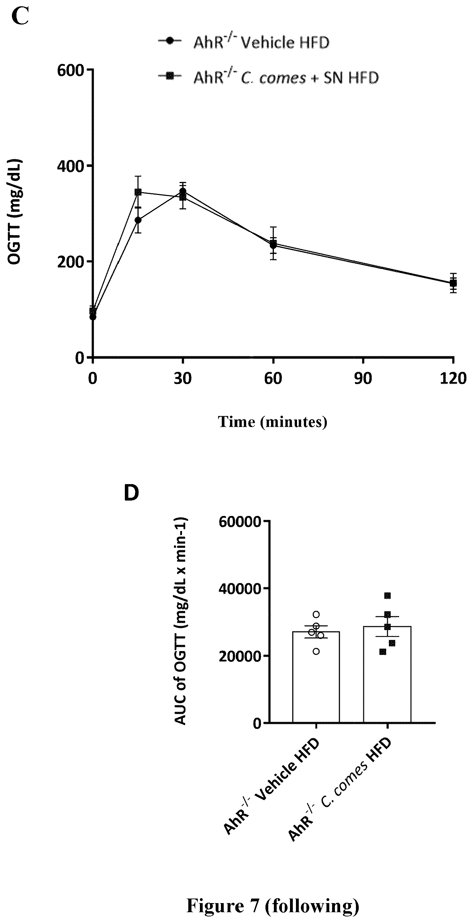

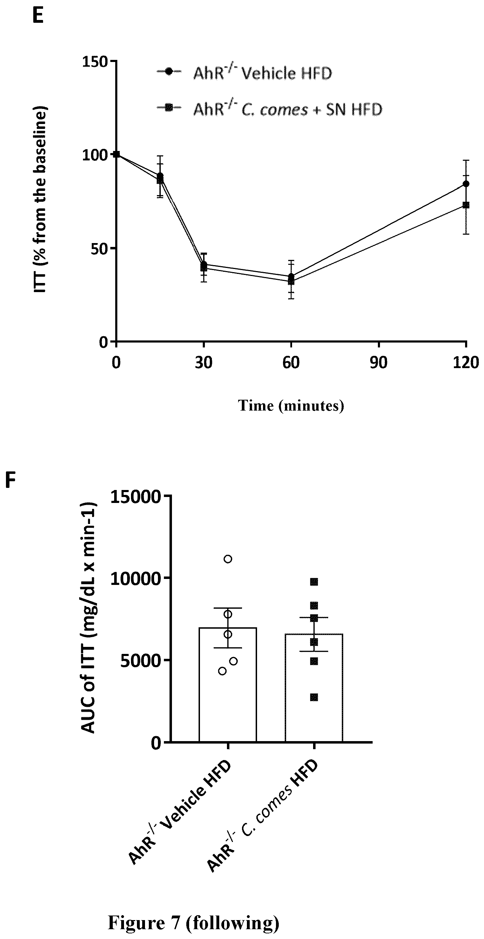

**Figure 7** : Supplementation of *Coprococcus comes* in AhR -/- mice is not sufficient to alleviate diet-induced metabolic impairments. **(A)** Body weight gain, **(B)** Food intake, (C) Blood glucose level after 16h of fasting of Conv- and HFD-fed mice supplemented with C. comes or vehicle. **(D)** AUC of the OGTT, **(E)** Blood glucose levels after 4h of fasting during the ITT, **(F)** AUC of the ITT.

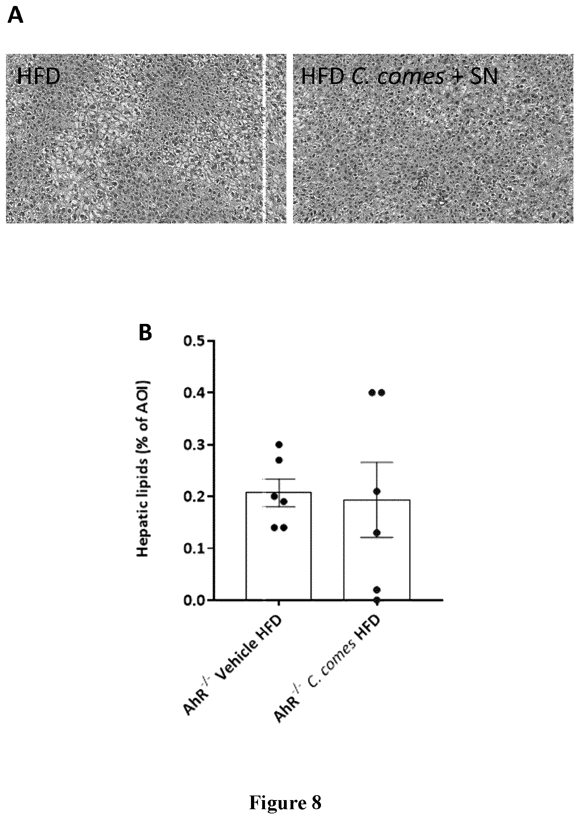

**Figure 8** : Supplementation of *Coprococcus comes* in AhR -/- mice has no impact on hepatic steatosis in HFD mice. **(A)** Representative pictures of H&E-stained liver sections from indicated mice. **(B)** Lipid area, calculated as the percent area of interest (AOI), in the liver cross-sections of Conv- and HFD-fed mice supplemented with C. comes or vehicle.

### DETAILED DESCRIPTION OF THE INVENTION

The inventors herein identified a bacterial probiotic associated with a high AhR activity. Indeed, they demonstrated that *Coprococcus comes* bacteria and/or their culture supernatant are able to protect mice from DSS-induced colitis, and to alleviate diet-induced metabolic impairments and to reduce nonalcoholic fatty liver disease (NAFLD) features such as hepatic steatosis in high-fat diet (HFD) mice. All these effects were shown to be AhR dependent.

Accordingly, in a first aspect; the present invention relates to a composition comprising *a Coprococcus* bacterium and/or a culture extract thereof exhibiting AhR agonist activity, for use in the treatment of a disease selected from the group consisting of metabolic syndrome and associated disorders being selected from the group consisting of cardiovascular diseases, insulin resistance, glucose intolerance, non alcoholic fatty liver disease and lipodystrophy, and inflammatory bowel diseases.

*Coprococcus* (Uniprot Taxon ID: 33042) is a genus of anaerobic cocci which are part of the human fecal microbiota. In preferred embodiments, the *Coprococcus* bacterium is *Coprococcus comes* (e.g. Uniprot Taxon ID: 410072). *Coprococcus comes* bacteria are commercially available, for example from ATCC^{®} collection (ATCC^{®} 27758^{™}).

*Coprococcus* bacteria can be cultured in anaerobic conditions, at 37°C and in a suitable medium. Examples of suitable media include, but are not limited to, LYHBHI medium (Brain-heart infusion medium supplemented with 0.5% yeast extract and 1% hemine) supplemented with cellobiose (1 mg/ml), maltose (1 mg/ml), cysteine (0.5 mg/ml), vitamin K1 (0.0002%) and vitamin K3 (0.0002%), ATCC^{®} Medium 1102 (Chopped meat carbohydrate medium with 0.1% cellobiose, 0.1% maltose, 0.1% starch, and 0.1% Tween 80) or ATCC^{®} Medium 260 (Trypticase soy agar/broth with defibrinated sheep blood).

*Coprococcus* bacteria for use according to the present invention exhibit AhR activation properties.

As used herein, the term "AhR" has its general meaning in the art and refers to Aryl hydrocarbon receptor, a transcription factor which is activated by diverse compounds and regulates the expression of xenobiotic metabolism genes. Aryl hydrocarbon receptor (AhR) is a member of the family of basic helix-loop-helix transcription factors, the bHLH-PAS (basic helix-loop-helix/Per-ARNT-Sim) family (Schmidt and Bradfield, 1996, Annu Rev Cell Dev Biol. 12, 55-89; Safe S et al. 2013, Toxicol Sci., 135,1-16). It is described in the Uniprot database under P35869. The sequences of reference in Genbank are the followings: NM_001612.1 and NP_001621.4.

The term "AhR activity" has its general meaning in the art and refers to the biological activity associated with the activation of the AhR resulting from its signal transduction cascade, and including any of the downstream biological effects resulting from the binding of an AhR agonist, e.g. a natural ligand, to AhR. Analyzing the AhR activation level may be assessed by any of a wide variety of well-known methods (Lehmann et al., 1995, Journal of Biological Chem., 270, 12953-12956; He et al., 2011, Environ Toxicol Chem, 30, 1915-1925; and Gao et al., 2009, Anal Biochem, 393, 163-175).

The term "AhR activation properties" or "AhR agonist activity" means potency in being able to induce AhR activity, i.e. to activate a signaling pathway that is initiated by AhR activation, and may involve any kind of activating mechanism. Therefore, it is not always necessary for a microbial body itself to be an AhR ligand, and for example a secretory substance produced by a microbe may have AhR-activating potency. The AhR activation properties of a bacterium may be assessed by cell-based assays such as described in the examples, He et al., 2011, supra and Gao et al., 2009, supra. In particular, the AhR activation level may be assessed by luciferase activity in AhR-responsive recombinant cells such as AhR-responsive recombinant guinea pig (G16L1.1c8), rat (H4L1.1c4), mouse (H1L1.1c2) and human (HG2L6.1c3) cells. The AhR activation level may also be assessed by measuring the ability to stimulate AhR-dependent gene expression using recombinant mouse hepatoma (Hepa1c1c7) cell-based CALUX (H1L1.1c2 and H1L6.1c2) clonal cell lines that contain a stably integrated AhR-/dioxin-responsive element (DRE)-driven firefly luciferase plasmid (pGudLuc1.1 or pGudLuc6.1, respectively) and CAFLUX (H1G1.1c3) clonal cell lines (He et al., 2011, supra). Typically, the AhR agonist activity is measured by performing the method described in the examples, i.e. by assessing luciferase activity in AhR-responsive recombinant cells, preferably H1L1.1c2 cells, in the presence of the bacterium to be tested or a culture extract thereof, in particular a culture supernatant thereof. In particular, the AhR agonist activity may be measured by AhR-dependent chemically activated luciferase expression (CALUX) analysis in recombinant H1L1.1c2 cells containing the stably integrated DRE-driven firefly luciferase reporter plasmid pGudLuc1.1, in the presence of the bacterium to be tested or a culture extract thereof, in particular a culture supernatant thereof, and an increase, preferably an increase of at least 2%, at least 5%, at least 10%, at least 20%, at least 50%, at least 75% or at least 100%, of the luciferase (or AhR) activity by comparison to a control sample without said bacterium or culture extract indicates that the bacterium exhibits AhR agonist activity. Preferably, the AhR agonist activity is measured by the method of described in the examples, in particular by assessing luciferase activity in AhR-responsive recombinant cells, preferably H1L1.1c2 cells, and 10% culture supernatant of a *Coprococcus* bacterium used in the invention increases AhR activity by at least 1.5-fold change by comparison with AhR activity obtained in a control sample without said culture supernatant.

The composition for use according to the invention may comprise one or several strains of *Coprococcus,* in particular one or several strains of *Coprococcus comes,* and/or a culture extract of one or several strains of *Coprococcus,* in particular a culture extract of one or several strains of *Coprococcus comes.*

Preferably, the composition of the invention comprises at least one *Coprococcus comes* bacterium and/or a culture extract thereof.

More preferably, the composition for use according to the invention comprises *Coprococcus comes* bacterium deposited under accession number DSM 33359 on November 21, 2019 at Deutsche Sammlung von Mikroorganismen und Zellkulturen GmbH (DSMZ, Inhoffenstr. 7B, D-38124 Braunschweig, Germany) or a functionally equivalent mutant thereof, and/or a culture extract thereof.

As used herein, the term "mutant" should be understood as a strain derived, or a strain which can be derived from a strain of the invention (or the mother strain) by means of e.g. genetic engineering, radiation and/or chemical treatment. The mutant can also be a spontaneously occurring mutant. The mutant is a functionally equivalent mutant, e.g. a mutant that has substantially the same, or improved, properties (in particular AhR activation properties) as the mother strain. Such a mutant is a part of the present invention. Especially, the term "mutant" refers to a strain obtained by subjecting a strain of the invention to any conventionally used mutagenization treatment including treatment with a chemical mutagen such as ethane methane sulphonate (EMS) or N-methyl-N'-nitro-N-nitroguanidine (NTG), UV light, or to a spontaneously occurring mutant. A mutant may have been subjected to several mutagenization treatments (a single treatment should be understood one mutagenization step followed by a screening/selection step), but it is presently preferred that no more than 20, or no more than 10, or no more than 5, treatments (or screening/selection steps) are carried out. In a presently preferred mutant, less than 1%, less than 0.1%, less than 0.01%, less than 0.001% or even less than 0.0001% of the nucleotides in the bacterial genome have been mutated, i.e. substituted, inserted or deleted, compared to the mother strain. Preferably, the genome of the mutant has at least 99% sequence identity to the genome of the mother strain and said mutant exhibits AhR agonist activity.

In an embodiment, the composition for use according to the invention comprises an inactivated *Coprococcus* bacterium, i.e. at least one inactivated *Coprococcus* strain. Preferably, said strain is a *Coprococcus comes* bacterium. More preferably, said strain is DSM 33359 or a mutant
thereof. In this embodiment, it is not excluded that the composition also comprises other bacterial or culture components such as living bacteria and/or cell debris and/or culture medium. Inactivated bacteria are not able to grow when cultured in a suitable medium. Preferably, inactivated bacteria are killed bacteria. Bacteria can be inactivated by any means known by the skilled person such as heat treatment. Inactivated bacteria can be preserved before administration by any method known by the skilled person such as lyophilization and subsequent storage, preferably at temperatures ranging from +4 °C to -80 °C.

In a preferred embodiment, the composition for use according to the invention comprises a living *Coprococcus* bacterium, i.e. at least one living *Coprococcus* strain. Preferably, said strain is a *Coprococcus comes* bacterium. More preferably, said strain is DSM 33359 or a mutant thereof. In this embodiment, it is not excluded that the composition also comprises other bacterial or culture components such as dead bacteria and/or cell debris and/or culture medium. Living bacteria may be preserved before administration by freezing with liquid nitrogen, gradual freezing or lyophilization and subsequent storage, preferably at temperatures ranging from +4 °C to -80 °C.

In another embodiment, the composition for use according to the invention comprises a culture extract of a *Coprococcus* bacterium, preferably a culture extract of a *Coprococcus comes* bacterium, more preferably a culture extract of DSM 33359 strain or a mutant thereof.

As used herein, the term "culture extract" refers an extract obtained from the culture of a *Coprococcus* bacterium, preferably a *Coprococcus comes* bacterium, more preferably DSM 33359 strain or a functionally equivalent mutant thereof, in a suitable cell culture medium and under suitable conditions, said extract exhibiting AhR agonist activity. The AhR agonist activity of the extract may be assessed as described above. The extract may be any fraction obtained from the culture or from bacterial cells, such as a culture supernatant, cell debris, cell walls, DNA or RNA extracts, protein extracts, and in general any preparation derived from bacterial cells or cell culture by chemical, physical and/or enzymatic treatments. The culture extract may be free of intact bacterial cells or may contain some residual intact bacterial cells, preferably less than 10³ cells per mL.

In preferred embodiments, the culture extract is a culture supernatant. As used herein, the term "culture supernatant" refers a supernatant obtained from growing a *Coprococcus* bacterium, preferably a *Coprococcus comes* bacterium, more preferably DSM 33359 strain or a functionally equivalent mutant thereof, in a suitable cell culture medium and under suitable conditions, said culture
supernatant exhibiting AhR agonist activity. In particular, this term refers to the liquid broth remaining when cells grown in a medium are separated from the culture medium by for example centrifugation, filtration, sedimentation, or other means well known in the art. Optionally, the culture supernatant may be further diluted, concentrated, dried and/or lyophilized. The culture supernatant may be free of living bacterial cells or may contain some residual living bacterial cells, preferably less than 10³ cells per mL. The culture supernatant may also comprise dead bacteria and/or cell components such as cell debris.

In a particular embodiment, the culture supernatant is obtained by growing *Coprococcus* bacterium, preferably *Coprococcus comes* bacterium, more preferably strain DSM 33359 or a functionally equivalent mutant thereof, in a suitable medium, separating bacterial cells from the culture medium, e.g. by centrifugation, filtration or sedimentation, and recovering the culture supernatant.

In another particular embodiment, the culture supernatant is obtained by growing *Coprococcus* bacterium, preferably *Coprococcus comes* bacterium, more preferably strain DSM 33359 or a functionally equivalent mutant thereof, in a suitable medium, lysing bacterial cells in the culture medium, and recovering the culture supernatant (comprising dead bacteria and/or cell debris, i.e. the lysate).

In a further particular embodiment, the culture supernatant is obtained by growing *Coprococcus* bacterium, preferably *Coprococcus comes* bacterium, more preferably strain DSM 33359 or a functionally equivalentt mutant thereof, in a suitable medium, lysing bacterial cells in the culture medium, separating bacterial cells and cell debris from the culture medium, e.g. by centrifugation, filtration or sedimentation, and recovering the culture supernatant.

Optionally, in any of these embodiments, the culture supernatant may be further diluted or concentrated. In some preferred embodiments, the culture supernatant is dried or lyophilized.

In a further embodiment, the composition comprises a *Coprococcus* bacterium, preferably *Coprococcus comes* bacterium, more preferably strain DSM 33359 or a functionally equivalent mutant thereof, and a culture extract thereof, preferably a culture supernatant thereof. Preferably, the composition comprises a living *Coprococcus* bacterium. In said embodiment, the composition may comprise a cell culture comprising a *Coprococcus* bacterium. As used herein, the term "cell culture" refers to the mix of bacterial cells and liquid broth used to culture these cells. In particular, the cell culture may be obtained by inoculating a suitable liquid broth with a living *Coprococcus* bacterium, incubating said mix in suitable conditions (in particular in anaerobic conditions), and recovering cell culture. Alternatively, the composition may comprise a *Coprococcus* bacterium, preferably a living *Coprococcus* bacterium, and a culture extract thereof, preferably a culture supernatant thereof, obtained by any method disclosed above, in particular obtained by growing *Coprococcus* bacterium in a suitable medium, and a) separating bacterial cells from the culture medium, e.g. by centrifugation, filtration or sedimentation, and recovering the culture supernatant; or b) lysing bacterial cells in the culture medium, and recovering the culture supernatant (comprising dead bacteria and/or cell debris, i.e. the lysate); or c) lysing bacterial cells in the culture medium, separating bacterial cells and cell debris from the culture medium, e.g. by centrifugation, filtration or sedimentation, and recovering the culture supernatant. Optionally, the culture supernatant may be further diluted, concentrated, dried and/or lyophilized.

In another embodiment, the composition comprises a *Coprococcus* bacterium, preferably a living *Coprococcus* bacterium, and a culture extract, preferably a culture supernatant, of another *Coprococcus* bacterium. Preferably, the *Coprococcus* bacterium and/or the *Coprococcus* bacterium used to obtained the culture extract is a *Coprococcus comes* bacterium, more preferably is DSM 33359 strain or a functionally equivalent mutant thereof.

In some particular embodiments, the composition does not comprise any *Coprococcus catus* bacterium or any culture extract thereof, and/or any *Coprococcus eutactus* bacterium or any culture extract thereof.

The composition may further comprise at least one additional active ingredient, in particular one or several additional bacterial probiotics and/or one or several AhR agonists.

The composition for use according to the invention may comprise one or several additional bacterial probiotics. The term "bacterial probiotic" has its general meaning in the art and refers to a useful bacterium that can bring a beneficial action to the host health, i.e. which is applicable to the prevention, treatment or cure of a disease or condition of the host, preferably a human being. This term may refer to a dead or living bacterium. Preferably, this term refers to a living bacterium.

Preferably, one or several of these additional bacterial probiotics exhibit AhR activation properties. The AhR may be activated by the bacterial probiotic as AhR ligand, by a secretory substance produced by said bacterial probiotic and having AhR-activating potency, or by a dead microbial body or homogenate of said bacterial probiotic.

Preferably, said one or several bacterial probiotics are capable of producing an AhR agonist. Such bacterial probiotics are selected from the group consisting of bacteria belonging to the genera *Allobaculum* (e.g. *Allobaculum stercoricanis), Adlercreutzia, Anaerostipes* (e.g. *Anaerostipes hadrus, Anaerostipes caccae* and *Anaerostipes butyraticus), Bifidobacterium, Propionibacterium, Bacteroides, Eubacterium, Enterococcus, Ruminococcus* (e.g. *Ruminococcus gnavus)* and *Faecalibacterium* (e.g. *Faecalibacterium prausnitzii), Escherichia coli,* and lactic acid bacteria such as bacteria belonging to the genera *Lactobacillus* (e.g. *Lactobacillus reuteri, Lactobacillus taiwanensis, Lactobacillus johnsonii, Lactobacillus animalis, Lactobacillus murinus, Lactobacillus salivarius, Lactobacillus gasseri, Lactobacillus bulgaricus,* and *Lactobacillus delbrueckii subsp. Bulgaricus)* and Streptococcus (e.g. *Streptococcus thermophilus).*In a particular embodiment, the composition may comprise one or several bacterial probiotics selected from the group consisting of bacteria belonging to the genera *Allobaculum, Adlercreutzia, Propionibacterium, Enterococcus, Escherichia coli, Lactobacillus* and *Streptococcus.*

In preferred embodiments, said one or several additional probiotics are selected from the group consisting of strains CNCM 1-5019 *(Lactobacillus taiwanensis),* CNCM I-5020 *(Lactobacillus murinus),* CNCM I-5021 *(Lactobacillus animalis*), CNCM I-5022 *(Lactobacillus reuteri)* and CNCM I-5023 *(Lactobacillus reuteri),* deposited on September 30, 2015 at the Collection Nationale de Cultures de Microorganismes (CNCM, Institut Pasteur, 25 rue du Docteur Roux, 75724 Paris Cedex 15, France), *Ruminococcus gnavus* ATCC 29149, *Lactobacillus salivarius* DSM 20555, *Lactobacillus reuteri* DSM 20016, *Lactobacillus gasseri* DSM 20243, *Faecalibacterium prausnitzii* A2-165, *Escherichia coli* MG1665, *Anaerostipes hadrus* DSM *3319, Anaerostipes caccae* DSM 14662, *Anaerostipes butyraticus* DSM 22094 and *Allobaculum stercoricanis* DSM 13633.

The composition for use according to the invention may also comprises one or several AhR agonists.

The AhR agonists are small organic molecules or peptides and include synthetic and naturally occurring compounds. The term "AhR agonist" has its general meaning in the art and refers to a compound that activates the AhR, preferably that selectively activates the AhR. AhR agonists may be a natural AhR ligand or any compound that can directly or indirectly stimulate the signal transduction cascade related to the AhR. As used herein, the term "selectively activates" refers to a compound that preferentially binds to and activates AhR with a greater affinity and potency, respectively, than its interaction with the other members of bHLH-PAS transcription factors family. Compounds that prefer AhR, but that may also activate other sub-types, as partial or full agonists are also contemplated. Tests and assays for determining whether a compound is an AhR agonist are well known by the person skilled in the art such as described in Ji et al., 2015, Dig Dis Sci, 60, 1958-1966; Furumatsu et al., 2011,, Dig Dis Sci, 56, 2532-2544; WO 2013/171696; WO 2012/015914; US 6,432,692. *In vitro* and *in vivo* assays may be used to assess the potency and selectivity of the candidate agents to induce AhR activity.

Examples of AhR agonists include, but are not limited to, halogenated aromatic hydrocarbons (e.g. polychlorinated dibenzodioxins, dibenzofurans and biphenyls), polycyclic aromatic hydrocarbons (e.g. 3-methylcholanthrene, benzo-α-pyrene, benzanthracenes and benzoflavones), indoles derivatives, kynurenine, kynurenic acid, tryptophan catabolites of the microbiota such as indole-3-aldehyde (lAld), indole propionic acid, tryptamine, indole 3-acetate, 3-indoxyl sulfate, 6-formylindolo(3,2-b)carbazole (FICZ), indolo(3,2-b)carbazole (ICZ), 2-(1'H-indole-3'-carbonyl)-thiazole-4-carboxylic acid methyl ester (ITE) or its precursor 2-(1'H-indole-3'-carbonyl)-thiazole-4-carboxylate (ITC), and analogs thereof disclosed in US 7,419,992, 2,3,7,8-tetrachlorodibenzo-p-dioxin (TCDD), polycyclic aromatic hydrocarbon (PAH), polychlorinated biphenyl (PCB), 3-indoxyl-sulfate (13S),1-(4-Methylphenyl)-2-(4,5,6,7-tetrahydro-2-imino-3(2H)-benzothiazolyl)ethanone hydrobromide (Pifithrin-α hydrobromide), (2'Z,3'E)-6-Bromo-1-methylindirubin-3'-oxime (MeB10), tryptophan derivatives such as indigo dye and indirubin, flavonoids, biphenyls, tetrapyrroles such as bilirubin, the arachidonic acid metabolites lipoxin-A4 and prostaglandin G, modified low-density lipoprotein and several dietary carotenoids (Denison et al., 2002, Chem. Biol. Interact. 141, 3-24; Denison et al., 2003, Annu. Rev. Pharmacol. Toxicol. 43, 309-334; Adachi J et al., 2001, J. Biol. Chem., 276, 31475-1478; Sinal C J and Bend J R, 1997, Mol. Pharmacol., 52, 590-9; Seidel S D, et al., 2001, J. Biochem. Mol. Toxicol., 15, 187-196; McMillan B J and Bradfield C A, 2007, Proc. Natl. Acad. Sci. U.S.A., 104, 1412-1417; Stevens et al., 2009, Immunology., 127, 299-311), AhR agonists disclosed in Bisson et al., 2009, J. Med. Chem, 52, 5635-5641 (e.g. 5-hydroxy-7-methoxyflavone, 7-methoxyisoflavone, 6-methylflavone, 3-hydroxy-6-methylflavone, pinocembrin (5,7-dihydroxyflavanone) and 7,8,2'-trihydroxyflavone), compound VAF347 [4-(3-chlorophenyl)-N-[4-(trifluoromethyl)phenyl]pyrimidin-2-amine], and its pro-drug version VAG539 [4-(3-chloro-phenyl)-pyrimidin-2-yl]-(4-trifluoromethyl-phenyl)-carbamic acid 2-[(2-hydroxy-ethyl)-methyl-amino]-ethyl ester] (Lawrence B P, 2008, Blood, 112, 1158-1165), semaxanib (SU5416) [3-(3,5-dimethyl-1H-pyrrol-2-ylmethylene)-1,3-dihydro-indole-2-one] (Mezrich J D, et al. (2012) PLoS ONE 7(9): e44547), selective AhR modulators (SAhRM) (e.g. diindolylmethane (DIM), methyl-substituted diindolylmethanes, dihalo- and dialkylDIM analogs, β-naphthoflavone (βNF) (5,6 benzoflavone (5,6 BZF) and moieties described, for example, in Safe et al., 2013, Toxicol Sci., 135, 1-16; Furumatsu et al., 2011,, Dig Dis Sci, 56, 2532-2544; and WO 2012/015914), compounds described in WO 2012/015914 (e.g. CB7950998), 1,4-dihydroxy-2-naphthoic acid (DHNA) and natural AhR Agonists (NAhRAs) disclosed in WO 2013/171696 and WO 2009/093207, Mexiletine, Nimidipine, Flutamide, Atorvastatin, Leflunomide, and Ginseng (Hu W et al, 2007, Mol Pharmacol., 71, 1475-86, O'Donnell EF, et al, 2010, PLoS One, 5(10). pii: e13128; Wang Y, et al, 2008, Eur J Pharmacol., 601, 73-78).

In a particular embodiment, said one or several AhR agonists are selected from the group consisting of approved drugs having an agonist effect on AhR, preferably from the group consisting of Mexiletine, Nimidipine, Flutamide, Atorvastatin, Leflunomide and Ginseng.

As additional active ingredients, the composition may also comprise one or several drugs useful in the treatment of metabolic syndrome and associated disorders, or useful in the treatment of inflammatory bowel diseases. Examples of drugs useful in the treatment of inflammatory bowel diseases include, but are not limited to, corticosteroids, 5-aminosalicylates, immunosuppressors such as cyclosporin, azathioprine, 6-mercaptopurine, methotrexate, anti-TNF agents (such as infliximab, adalimumab, golimumab, certolizumab), , anti-integrin agents (such as natalizumab, vedolizumab) anti-IL12 and/or -IL23 antibodies (such as ustekinumab), JAK inhibitors (such as tofacitinib), antibiotics, anti-diarrheals, pain relievers, iron supplements, vitamin B-12, calcium and vitamin D.

In a particular embodiment, the composition consists essentially in one or several *Coprococcus* bacteria, preferably one or several living *Coprococcus* bacteria, and/or culture extract thereof, preferably culture supernatant thereof. As used herein, the term "consists essentially in" is intended to refer to a composition that does not comprise any other active ingredient, in particular does not comprise any other bacterium, especially any other bacterial probiotic.

In preferred embodiments, the composition for use according to the invention is a pharmaceutical composition comprising a *Coprococcus* bacterium as defined above, preferably a living *Coprococcus* bacterium, and/or a culture extract thereof, preferably a culture supernatant thereof, and a pharmaceutically acceptable excipient.

As used herein, the term "pharmaceutically acceptable" refer to molecular entities and compositions that do not produce an adverse, allergic or other untoward reaction when administered to a mammal, especially a human, as appropriate. A pharmaceutically acceptable excipient refers to a non-toxic solid, semi-solid or liquid filler, diluent, encapsulating material or formulation auxiliary of any type. The pharmaceutically acceptable excipients that can be used in the composition for use according to the invention are well known to the skilled person and may vary according to the disease to be treated and the administration route.

The composition for use according to the invention can be administered by any method suitable for depositing in the gastrointestinal tract, preferably the small intestine and/or the colon, of the subject to be treated. In particular, the composition can be administered by enteral or parenteral route, preferably by oral, sublingual, subcutaneous, intramuscular, intravenous, transdermal, local or rectal administration route. Preferably, the composition of the invention is administered, or is adapted to be administered, by rectal or oral route.

In an embodiment, the pharmaceutical composition is to be administered by oral route. For oral administration, the pharmaceutical composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatin, sugars such as lactose or dextrose, and natural or synthetic gums can be used as binders. Disintegrants may also be necessary in the tablets to facilitate break-up of the tablet. Disintegrants include starches, clays, celluloses, algins, gums and crosslinked polymers. Moreover, lubricants and glidants may also be included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. Well-known thickening agents may also be added to compositions such as corn starch, agar, natural or synthetic gums, resins, methylcellulose, sodium carboxymethylcellulose, guar, xanthan and the like. Preservatives may also be included in the composition, including methylparaben, propylparaben, benzyl alcohol and ethylene diamine tetraacetate salts.

In some particular embodiments, the composition may be a beverage or drink composition, a food composition or a feedstuff composition.

Preferably, for oral administration, the composition is in a gastro-resistant oral form allowing the active compounds contained in the composition, to pass the stomach and be released into the intestine. The material that can be used in enteric coatings includes, for example, alginic acid, cellulose acetate phthalate, plastics, waxes, shellac and fatty acids (e.g. stearic acid or palmitic acid).

In another embodiment, the pharmaceutical composition is to be administrated by rectal route. Suitable rectal-route forms include, but are not limited to, suppository and enema. In particular, the active compounds can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Compositions according to the invention may be formulated to release the active ingredients substantially immediately upon administration or at any predetermined time or time period after administration.

The composition for use according to the invention is used in the treatment of a disease selected from the group consisting of metabolic syndrome and associated disorders being selected from the group consisting of cardiovascular diseases, insulin resistance, glucose intolerance, non alcoholic fatty liver disease and lipodystrophy, and inflammatory bowel diseases.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of the disease or symptoms associated with it. In other embodiments, this term refers to minimizing the spread or worsening of the disease resulting from the administration of one or more therapeutic agents, e.g. the composition of the invention, to a subject with such a disease.

In some embodiments, the disease is the metabolic syndrome or any associated disorder. The metabolic syndrome is defined by a clustering of at least three of the five following medical conditions:
- Abdominal (central) obesity;
- Elevated blood pressure;
- Elevating fasting plasma glucose;
- High serum triglycerides; and
- Low high-density lipoprotein (HDL) levels.

It is important to note that obesity doesn't equate to metabolic syndrome. Patients who are of normal weight may also have a metabolic syndrome and, on the opposite, obese people may not have a metabolic syndrome. Indeed, as mentioned above, the metabolic syndrome is established if the obesity is an abdominal obesity and if at least two other medical conditions are observed.

According to the International Diabetes Federation, a consensus worldwide definition of the metabolic syndrome (2006) is: Central obesity (defined as waist circumference with ethnicity-specific values) and any two of the following:
- Elevated blood pressure (BP): systolic BP > 130 or diastolic BP >85 mm Hg, or treatment of previously diagnosed hypertension;
- Elevating fasting plasma glucose (FPG): >100 mg/dL (5.6 mmol/L), or previously diagnosed type 2 diabetes;
- High serum triglycerides refers to > 150 mg/dL (1.7 mmol/L) or specific treatment for this lipid abnormality.
- Low high-density lipoprotein (HDL) levels: < 40 mg/dL (1.03 mmol/L) in males, < 50 mg/dL (1.29 mmol/L) in females, or specific treatment for this lipid abnormality.

If BMI is >30 kg/m², central obesity can be assumed and waist circumference does not need to be measured.

Metabolic syndrome is associated with the risk of developing other disorders, i.e. associated disorders. The associated disorders are selected from the group consisting of cardiovascular diseases, in particular coronary heart disease, especially heart attack and stroke, insulin resistance, glucose intolerance, non alcoholic fatty liver disease, especially nonalcoholic steatohepatitis, and lipodystrophy.

In some other embodiments, the disease is an inflammatory bowel disease. As used herein, "inflammatory bowel diseases" or "IBD" refers to any of a variety of diseases characterized by inflammation of all or part of the intestines. Examples of inflammatory bowel diseases include, but are not limited to, Crohn's disease (such as enteritis, ileitis, colitis, ileocolitis, gastroduodenal and perianal Crohn's), ulcerative colitis (such as ulcerative enterocolitis, ulcerative ileocolitis, ulcerative proctitis, ulcerative rectosigmoiditis, pseudopolyposis of colon, mucosal proctocolitis, and left-sided or extensive ulcerative colitis), indeterminate colitis (IC), other noninfective gastroenteritis, enteritis, enterocolitis and colitis (such as non-microscopic colitis including collagenous colitis and lymphocytic colitis, radiation-induced, toxic-induced, allergic-induced, dietetic-induced, ischemic, eosinophilic gastroenteritis, enteritis, enterocolitis or colitis, segmental colitis associated with diverticula, diversion colitis and Behcet's colitis) as well as pouchitis. In some particular embodiments, the inflammatory bowel disease is not antibiotic associated colitis or more particularly is not *Clostridium difficile* associated diarrhea.

Preferably, the inflammatory bowel disease is selected from the group consisting of Crohn's disease and ulcerative colitis, more preferably is ulcerative colitis.

The subject to be treated with the composition for use according to the invention is an animal, preferably a mammal, even more preferably a human, including adult, child, newborns and human at the prenatal stage. As used herein, the terms "subject", "individual" and "patient" are interchangeable.

In an embodiment, the subject exhibits a decreased AhR activity, especially in a feces sample, more particularly a decreased AhR agonist activity of the gut microbiota. In a particular embodiment, the activity of AhR is measured for the subject. Preferably, the AhR activity is the activity of the microbiota and is measured in a feces sample.

In a preferred embodiment, the subject exhibits a decreased AhR activity by comparison with a predetermined reference value. As used herein, the "reference value" refers to a threshold value or a cut-off value. The reference value can be determined experimentally, empirically, or theoretically. Preferably, the reference value is derived from the AhR activation level determined in one or several feces samples derived from one or more healthy subjects, especially who are not suffering of a metabolic syndrome, an associated disorder and/or IBD. Preferably, the subject having a decreased AhR activity exhibits a AhR activity which is at least 10% lower than the predetermined reference value, more preferably at least 20%, 30%, or 50% lower than the predetermined reference value.

The dosage of *Coprococcus* bacterial cells and/or culture extract may be appropriately adjusted according criteria such as age, symptoms, body weight, and intended application such as to obtain a therapeutically efficient amount. The term "therapeutically efficient amount" as employed herein refers to the amount necessary for having a beneficial impact on the disease to be treated, i.e. to prevent, remove or reduce at least one deleterious effect of the disease.

In embodiments wherein the disease to be treated is metabolic syndrome or an associated disorder, an therapeutically efficient amount is preferably defined as the amount necessary for having an impact (i.e. i.e. to prevent, remove or reduce) on one of the five medical conditions defining the metabolic syndrome:
- Abdominal (central) obesity (TOF1);
- Elevated blood pressure;
- Elevating fasting plasma glucose;
- High serum triglycerides; and
- Low high-density lipoprotein (HDL) levels.

In addition or alternatively, the therapeutically efficient amount may be defined as the amount necessary for having an impact on the insulin sensitivity, the glucose tolerance, the weight gain and/or the intestinal inflammation due to HFD. In a preferred embodiment, the therapeutically efficient amount has an impact on several of these conditions.

In embodiments wherein the disease to be treated is IBD, an therapeutically efficient amount is preferably defined as the amount necessary for having an impact on intestinal inflammation or any symptom of the disease such as diarrhea, fever or pain.

In some embodiments, the composition for use according to the invention may comprise from 10³ to 10¹¹ living *Coprococcus* bacterial cells, preferably *Coprococcus comes* bacterial cells, more preferably bacterial cells of DSM 33359 or a functionally equivalent mutant thereof, per mg of composition and/or from 0.001 mg to 1000 mg of dried or lyophilized culture supernatant per g of composition.

In particular, the amount of *Coprococcus* bacteria, preferably *Coprococcus comes* bacteria, more preferably DSM 33359 or a functionally equivalent mutant thereof, ingested per day may be from 0.01×10¹¹ to 100×10¹¹ cells/body, preferably 0.1×10¹¹ to 10×10¹¹ cells/body, and more preferably 0.3×10¹¹ to 5×10¹¹ cells/body.

The content of *Coprococcus* bacteria, preferably *Coprococcus comes* bacteria, more preferably DSM 33359 or a functionally equivalent mutant thereof, contained in an orally ingested composition of the invention may be for example from 1% to 100% (w/w, i.e. bacteria dry weight/total dry weight of the composition), preferably from 1% to 75% (w/w), and more preferably from 5% to 50% (w/w).

The composition for use according to the invention may be administered as a single dose or in multiple doses.

In some embodiments, the composition is to be administered regularly, preferably between every day and every month, more preferably between every day and every two weeks, more preferably between every day and every week. In some particular embodiments, the composition is to be administered every day.

The duration of treatment with the composition of the invention may be comprised between 1 day and several years, preferably between 1 day and one year, more preferably between 1 day and 6 months.

Although not part of the claimed invention, the disclosure also relates to a method of (i) selecting a subject suffering of a disease selected from metabolic syndrome and associated disorders, and inflammatory bowel diseases, for a treatment with a composition of the invention or (ii) determining whether a subject affected with a disease selected from metabolic syndrome and associated disorders, and inflammatory bowel diseases, is susceptible to benefit from a therapy with a composition of the invention, wherein a decreased AhR activity in the subject, especially in a feces sample from the subject, indicates that a therapy with the composition is suitable. More particularly, the method comprises the steps of: i) determining the AhR agonist activity of the microbiota in a feces sample obtained from the subject, ii) comparing the level determined at step i) with a predetermined reference value, and iii) selecting the subject as suitable for the treatment when the level determined at step i) is lower than the predetermined reference value. The predetermined reference value is preferably a reference value derived from the AhR activation level determined in one or several feces samples derived from one or more healthy subjects, especially who are not suffering of a metabolic syndrome, an associated disorder and/or IBD.

The AhR activation level of the microbiota in a feces sample obtained from the subject may be assessed by any method described above. Additionally or alternatively, the AhR activation level of the microbiota in a feces sample obtained from the subject may be assessed by measuring tryptophan metabolism, i.e. by measuring Tryptophan (Trp), kynurenine (Kyn) and indole-3-acetic acid (IAA) concentrations (or other tryptophan metabolites), and optionally calculating Kyn/Trp, IAA/Trp and Kyn/IAA concentration ratios. Additionally or alternatively, the AhR activation level may also be assessed using colon samples obtained from the subject by analyzing the expression of AhR target genes (such as interleukins IL-22 and IL-17), measuring IL-17⁺ and IL-22⁺ cells number, measuring AhR and chaperone proteins heterodimerization, measuring AhR nuclear translocation, or measuring AhR binding to its dimerization partner (AhR nuclear translocator (ARNT)).

Preferably, the subject is selected for the treatment with the composition if the level determined at step i) is at least 10% lower than the predetermined reference value, more preferably at least 20%, 30%, or 50% lower than the predetermined reference value.

In another aspect, the present invention also relates to *Coprococcus comes* strain deposited with the Deutsche Sammlung von Mikroorganismen und Zellkulturen (DSMZ) under accession number DSM 33359, or a functionally equivalent mutant thereof. The present invention also relates to *Coprococcus comes* strain deposited with the Deutsche Sammlung von Mikroorganismen und Zellkulturen (DSMZ) under accession number DSM 33359, or a functionally equivalent mutant thereof, for use in the treatment of a disease selected from the group consisting of metabolic syndrome and associated disorders being selected from the group consisting of cardiovascular diseases, insulin resistance, glucose intolerance, non alcoholic fatty liver disease and lipodystrophy, and inflammatory bowel diseases.

### EXAMPLES

### Methods

### Mice

Male and female C57BL/6JRj mice were purchased from Janvier (France). *AhR*^{-/-} mice on the C57BL/6JRj background were obtained from the Jackson laboratory (JAX stock #002831) (Fernandez-Salguero et al. Science. 1995, 268, 722-726) and were bred at Saint-Antoine Research Center. //*22*^{-/-} mice (Kreymborg et al., J. Immunol. 2007, 179, 8098-8104 ) were obtained and bred at the Transgenose Institute (TAMM-CNRS, Orleans, France). All the mice were housed at the IERP facility (INRA, Jouy-en-Josas, France), which is accredited by the French "Direction Départementale de la Protection des Populations (DDPP78)". All experiments were performed in accordance with the Comite d'Ethique en Experimentation Animale (COMETHEA C2EA - 45, Jouy en Josas, France).

### Bacterial strain and growth conditions

*Coprococcus comes* was grown at 37°C in LYHBHI medium (Brain- heart infusion medium supplemented with 0.5% yeast extract (Difco) and 1% hemine (Sigma-Aldrich)) supplemented with cellobiose (1 mg/ml; Sigma-Aldrich), maltose (1 mg/ml; Sigma-Aldrich), cysteine (0.5 mg/ml; Sigma-Aldrich), vitamin K1 (0.0002%; Sigma-Aldrich) and vitamin K3 (0.0002%; Sigma-Aldrich) in an anaerobic chamber (0ppm O₂; 2% H₂). Different conditions of bacteria were used in both models, DSS treatment and metabolic syndrome : bacteria only, bacteria supplemented with the supernatant, supernatant or killed bacteria. After 24h of incubation, *Coprococcus comes* culture was centrifuged at 6,000g during 10 min at 4°C. Supernatant was harvested and supplemented with 16% of glycerol for better preservation at -80°C. The pellets were resuspended in complete LYHBHI medium supplemented with 16% of glycerol or with supernatant also supplemented with 16% of glycerol. Killed bacteria were obtained after boiling at 100°C during 30 min, then centrifugation (6,000g, 10 min, 4°C) was performed and the pellet was resuspended in complete LYHBHI medium supplemented with 16% of glycerol. Mice were inoculated daily with 10⁹ CFU of *Coprococcus comes* or vehicle (complete LYHBHI medium supplemented with 16% of glycerol) throughout the protocols.

### AhR/luciferase reporter assay

The H1L1.1c2 cell line was used as described before (Lamas et al., 2016, Nat Med. Jun;22(6):598-605). Control (2, 10 or 20% LYHBHI), supernatant of 24h bacteria culture were used and incubated with the H1L1.1c2 cell line during 24h. AhR activity was calculated by subtracting the luminescence of the control (2, 10 or 20% LYHBHI) from the luminescence obtained with the samples multiplied by the cytotoxicity value (LDH dosage).

### DSS model

C57BL/6JRj from Janvier lab, 7 weeks old, were housed in our specific pathogen free animal facility at IERP (INRA, Jouy en Josas, France). Five to ten female mice per group were used in all experiment, were maintained in a temperature controlled (23°C) facility with a strict 12h light/dark cycles and were given free access to food and water. After one acclimation week, we changed water with DSS 2% (MP Biomedical) during 7 days (day 0 to day 7), and a recovery period was practiced until day 12 (during 5 days). Body weight loss and DAI (disease activity index) were performed daily. After 12 days the samples were harvested.

| **Body weight loss** | | | | |
|---|---|---|---|---|
| 0 | | No loss | | |
| 1 | | 1-5% loss of body weight | | |
| 2 | | 6-10% loss of body weight | | |
| 3 | | 10-20% loss of body weight | | |
| 4 | | >20% loss of body weight | | |
| | | | | |

| **Stool consitency** | | | | |
|---|---|---|---|---|
| 0 | | Normal Feces | | |
| 1 | | Loose stool | | |
| 2 | | Watery diarrhea | | |
| 3 | | Slimy diarrhea, little blood | | |
| 4 | | Severe watery diarrhea with blood | | |
| | | | | |

| **Blood in stool** | | | | |
|---|---|---|---|---|
| 0 | | No blood | | |
| 1 | | Presence of blood | | |
| 2 | | Visible blood - low quantity | | |
| 3 | | Visible blood - medium quantity | | |
| 4 | | Visible blood - high quantity | | |

### Colon histology

Colon distal part (cut in three parts) was harvested at day 12 after DSS model and put directly in PFA (Rothi^{®}- Histofix 4%) during 48h and transferred in ethanol 70% until the standard pre-fixation automat and paraffin embedded. HES coloration was practiced on 5µm cut. Scoring was performed on the worst cut and scored as previously described (Sokol et al., Gastroenterology. 2013 Sep;145(3):591-601.e3.).

### MLN stimulation

MLN (mesenteric lymph node) cells was harvested and grinded on 40µM cells strainer. Cells were resuspended in complete RPMI (10% SVF, Pen/Strep) and counted with Accuri cytometer (BD). Two millions of cells were put on 24 wells plate and stimulated with PMA (50ng/ml, *Sigma)* and ionomycin (750ng/ml, *Sigma)* during 48h at 37°C. Supernatants of stimulation were recovered and ELISA *(Mabtech)* against IL-17A, IL-10, IFN-y and IL-22 were performed according to the manufacturer's instructions.

### Real time PCR

Samples were extracted with Rneasy Mini kit (Qiagen). Total RNA (1 µg) was reverse transcribed using LunaScript^{™} Reverse Transcription SuperMix Kit *(Biolabs).* The mRNA levels for the genes of interest were examined by quantitative RT-PCR using the Luna^{®} Universal qPCR Master Mix *(Biolabs)* or Universal Probe qPCR Master Mix *(Biolabs)* according to the manufacturer's protocol. Primers and probes used were obtained from Qiagen or Thermofisher: Cyp1a1 (TaqMan, *Thermofisher,* Mm00487218_m1), IL-22 (TaqMan, *Thermofisher,* Mm01226722_g1), Reg3γ (TTCCTGTCCTCCATGATCAAAA (SEQ ID NO: 1) / CATCCACCTCTGTTGGGTTCA (SEQ ID NO: 2)), Reg3β (ATGCTGCTCTCCTGCCTGATG (SEQ ID NO: 3) / CTAATGCGTGCGGAGGGTATATTC (SEQ ID NO: 4)), AhRR (GGAGTCTCTCAATGGCTTCG (SEQ ID NO: 5) / CCGAGTACTCTGAGGGCAAG (SEQ ID NO: 6)). Relative levels of mRNA expression were normalized to HPRT1 (QT00166768) mRNA levels using a comparative method (2-ΔΔCt). Non-reverse-transcribed RNA samples and water were included as negative controls.

### Metabolic syndrome

C57BL/6JRj from Janvier lab, 5 weeks old, were housed in our specific pathogen free animal facility at IERP (INRA, Jouy en Josas, France). Six to eight male mice per group were used in all experiment, were maintained in a temperature controlled (23°C) facility with a strict 12h light/dark cycles and were given free access to food and water. Male C57BL/6JRj mice at 5 weeks of age were fed *ad libitum* with a purified control diet (Conv, Envigo MD.120508) or high fat diet (38% kcal fat, dominantly milk fat, Envigo MD.97222) for 12 weeks. Animals were weighed weekly, and weekly food consumption was measured in each cage. All animals were fasted during the night before the sacrifice and then euthanized by cervical dislocation, and appropriate tissues were harvested.

### Oral glucose tolerance test

OGGT was performed 3-7 days before sacrifice. Mice were fasted by removal of the food and bedding during the night before the experiment. After 15-16 hours of fasting, a glucose solution (2 g/kg per mice) was administered by oral gavage. Blood glucose levels at time 0 (fasting glucose, taken before glucose gavage) and 15, 30, 60 and 120 min after glucose gavage were analyzed using a OneTouch glucometer (Roche). The glucose level was plotted relative to time, and the AUC was calculated according to the trapezoidal rule.

### Intraperitoneal insulin tolerance test

ITT was performed 3-7 days before sacrifice. Mice were fasted by removal of the food and bedding 4 hours before the beginning of the experiment. After 4 hours of fasting, an insulin solution (0.5 U/kg) was administered intraperitoneally. Blood glucose levels at time 0 (fasting glucose, taken before glucose gavage) and at 15, 30, 60 and 120 min after the insulin challenge were analyzed using a OneTouch glucometer (Roche). The glucose levels were plotted relative to time, and the AUC was calculated according to the trapezoidal rule.

### Measurements of plasma parameters

Blood samples were collected in heparin-coated tubes via cardiac puncture and centrifuged. The plasma samples were then stored at -80°C until further analysis. Measurements of plasma cholesterol, high-density lipoprotein (HDL) were performed by using the Biochemistry Platform (CRI, UMR 1149, Paris) with an Olympus AU400 Chemistry Analyzer.

### Liver histology and hepatic triglyceride measurement

A slice of the left lobe of the liver was fixed in 4% PFA for 48 h and then transferred to ethanol, fixed in paraffin, trimmed, processed, sectioned into slices that were approximately 5 µm thick, mounted on a glass slide and stained with HES. Hepatic lipids were evaluated and quantified blindly by using the ImageJ software as previously described (Crane et al., 2015, Nat Med 21, 166-172 ; Schneider et al., 2012, Nat Methods 9, 671-675).

### Statistical analysis

Data were analyzed using Prism version 7 (Graphpad Software, San Diego, USA). The non-parametric Mann Withney test or the parametric one-way ANOVA tests with multiple Bonferroni's comparison tests were performed. Values are expressed as mean ± SEM. Statistical significance was defined at a p-value **** <0.0001, *** <0.001, ** <0.01, * <0.05.

### Results

### Coprococcus comes supernatant is associated with a high AhR activity

The inventors assayed the capacity of several bacterial strains to produce AhR agonists. Different supernatant of bacteria have been tested on H1L1.1c2, an AhR reporter cell line and they found that *Coprococcus comes* supernatant is associated with a high AhR activity (data not shown).

### C. comes supernatant protects from DSS-induced colitis

DSS-induced colitis mice were orally treated with C. *comes* (bacteria + supernatant, supernatant or killed bacteria). After 12 days of DSS-induced colitis, body weight loss, disease activity index (DAI), colon length and histological score were assessed (Figure 1). It was found that the administration of C. *comes* and/or its supernatant protect from DSS-induced colitis. In particular, C. comes supernatant was shown to induce a significant reduction of the production of IL-17, a pro-inflammatory cytokine, after MLN cells stimulation.

### C. comes protection against DSS-induced colitis is AhR dependent.

The inventors demonstrated that C. comes supernatant is able to activate AhR *in vitro* in H1L1.1c2 cells (Figure 2A). This result was confirmed in vivo using WT mice after C. *comes* supernatant gavage during 3 weeks. Activation of AhR was assessed by measuring expression of Cyp1a P450 cytochrome and AhR repressor (AhRR) in colon and liver by qPCR. An increase of these expression levels in colon of mice treated with *C*. *comes* supernatant indicates that the supernatant is able to activate AhR *in vivo* (Figure 2B).

In order to confirm that the protection is due to AhR activation, body weight loss, DAI, colon length and histological score after DSS-induced colitis were measured in AhR -/- mice with or without C. *comes* supernatant gavage (Figures 2C-G). No difference was observed between the group treated with the negative control and the group treated with C. *comes* supernatant. The AhR-dependent mechanism of the *C*. *comes* was thus confirmed by its lack of efficacy to protect AhR-/- mice from DSS-induced colitis.

### C. comes protection passes though IL-22 secretion

After 3 weeks of *C*. *comes* supernatant gavage, expression levels of Reg3y, Reg3β and IL-22 were measured in colon of WT mice. It was observed that expression of IL-22 as well as Reg3γ and Reg3β, two genes involved in IL-22 pathway, was significantly increased by the administration of *C*. *comes* supernatant (Figure 3A).

In order to confirm that the protection involves Il-22 pathway, body weight loss, DAI, colon length and histological score were assessed in IL-22^{-/-} mice after 9 days of DSS-induced colitis (Figures 3B-F). No difference was observed between the group treated with the negative control and the group treated with *C*. *comes* supernatant. This lack of efficacy to protect IL22^{-/-} mice from DSS-induced colitis confirms that *C*. comes protection passes though IL-22 secretion.

### Treatment with Coprococcus comes alleviates diet-induced metabolic impairments

Diet-induced mice models of metabolic syndrome (HFD-fed mice) were orally treated with C. *comes* (bacteria + supernatant, supernatant or killed bacteria) during 12 weeks.

It was found that *Coprococcus comes* supplementation reduces body weight gain independently of food intake (Figures 4A-B). HFD-fed mice supplemented with *Coprococcus comes* also showed better glucose clearance during oral glucose tolerance test (OGGT) (Figures 4C-D) and insulin sensitivity during insulin tolerance test (ITT) (Figures 4E-F). *Coprococcus comes* supplementation also reduces features of hepatic steatosis including lower hepatic lipids (Figure 5) and lower serum concentrations of cholesterol and HDL (Figure 6).

### C. comes protection against metabolic syndrome is AhR dependent.

Supplementation of *Coprococcus comes* in AhR -/- mice was shown to be not sufficient to alleviate diet-induced metabolic impairments. Indeed, in AhR -/- mice, *Coprococcus comes* supplementation does not have any impact on body weight gain, glucose and insulin tolerance (Figure 7) as well as features of hepatic steatosis (Figure 8). This lack of efficacy to treat metabolic syndrome in HFD-fed AhR-/- mice demonstrates the AhR-dependent mechanism of *C. comes.*

## Claims

1. A composition comprising a *Coprococcus* bacterium and/or a culture extract thereof exhibiting AhR agonist activity, for use in the treatment of a disease selected from the group consisting of metabolic syndrome and associated disorders being selected from the group consisting of cardiovascular diseases, insulin resistance, glucose intolerance, non alcoholic fatty liver disease and lipodystrophy, and inflammatory bowel diseases.

2. The composition for use according to claim 1, wherein the bacterium is a *Coprococcus comes* bacterium.

3. The composition for use according to claim 2, wherein the bacterium is *Coprococcus comes* strain deposited with the Deutsche Sammlung von Mikroorganismen und Zellkulturen (DSMZ) under accession number DSM 33359, or a functionally equivalent mutant thereof.

4. The composition for use according to any of claims 1 to 3, wherein the *Coprococcus* bacterium is a living *Coprococcus* bacterium.

5. The composition for use according to any of claims 1 to 4, wherein the culture extract is a culture supernatant.

6. The composition for use according to claim 5, wherein said composition comprises a living *Coprococcus* bacterium and a culture supernatant thereof, preferably a living *Coprococcus comes* bacterium and a culture supernatant thereof.

7. The composition for use according to any of claims 1 to 6, wherein the composition further comprises one or several additional bacterial probiotics capable of producing an AhR agonist and selected from the group consisting of bacteria belonging to the genera *Allobaculum, Adlercreutzia, Anaerostipes, Bifidobacterium, Propionibacterium, Bacteroides, Eubacterium, Enterococcus, Ruminococcus* and *Faecalibacterium, Escherichia coli,* and lactic acid bacteria such as bacteria belonging to the genera *Lactobacillus* and *Streptococcus,* preferably selected from the group consisting of bacteria belonging to the genera *Allobaculum, Adlercreutzia, Propionibacterium, Enterococcus, Escherichia coli, Lactobacillus* and *Streptococcus.*

8. The composition for use according to any of claims 1 to 7, wherein the composition further comprises one or several additional bacterial probiotics selected from the group consisting of strains available under CNCM deposit numbers CNCM I-5019, CNCM I-5020, CNCM I-5021, CNCM I-5022 and CNCM I-5023, *Ruminococcus gnavus* ATCC 29149, *Lactobacillus salivarius* DSM 20555, *Lactobacillus reuteri* DSM 20016, *Lactobacillus gasseri* DSM 20243, *Faecalibacterium prausnitzii* A2-165, *Escherichia coli* MG1665, *Anaerostipes hadrus* DSM 3319, *Anaerostipes caccae* DSM 14662, *Anaerostipes butyraticus* DSM 22094 and *Allobaculum stercoricanis* DSM 13633.

9. The composition for use according to any of claims 1 to 6, wherein the composition does not comprise any additional bacterium.

10. The composition for use according to any of claims 1 to 9, wherein said composition is to be administered by oral or rectal route.

11. The composition for use according to any of claims 1 to 10, wherein the disease is metabolic syndrome.

12. The composition for use according to any of claims 1 to 10, wherein the disease is a disorder associated to metabolic syndrome, said disorder being selected from the group consisting of cardiovascular diseases, insulin resistance, glucose intolerance, non alcoholic fatty liver disease and lipodystrophy.

13. The composition for use according to any of claims 1 to 10, wherein the disease is an inflammatory bowel disease, preferably selected from the group consisting of Crohn's disease, ulcerative colitis, indeterminate colitis (IC), other noninfective gastroenteritis, enteritis, enterocolitis and colitis, and pouchitis.

14. The composition for use according to any of claims 1 to 10, wherein the disease is an inflammatory bowel disease selected from Crohn's disease and ulcerative colitis.

15. *Coprococcus comes* strain deposited with the Deutsche Sammlung von Mikroorganismen und Zellkulturen (DSMZ) under accession number DSM 33359, or a functionally equivalent mutant thereof.

## Patentansprüche

1. Zusammensetzung, umfassend ein Coprococcus-Bakterium und/oder einen Kulturextrakt davon mit AhR-agonistischer Aktivität, zur Behandlung einer Erkrankung, ausgewählt aus der Gruppe bestehend aus metabolischem Syndrom und damit verbundenen Störungen, ausgewählt aus der Gruppe bestehend aus Herz-Kreislauf-Erkrankungen, Insulinresistenz, Glukoseintoleranz, nichtalkoholischer Fettlebererkrankung und Lipodystrophie sowie entzündlichen Darmerkrankungen.

2. Die Zusammensetzung zur Verwendung nach Anspruch 1, wobei es sich bei dem Bakterium um ein Coprococcus-comes-Bakterium handelt.

3. Die Zusammensetzung zur Verwendung nach Anspruch 2, wobei es sich bei dem Bakterium um einen Coprococcus-comes-Stamm handelt, der bei der Deutschen Sammlung von Mikroorganismen und Zellkulturen (DSMZ) unter der Zugangsnummer DSM 33359 hinterlegt ist, oder um eine funktionell äquivalente Mutante davon.

4. Die Zusammensetzung zur Verwendung nach einem der Ansprüche 1 bis 3, wobei es sich bei dem Coprococcus-Bakterium um ein lebendes Coprococcus-Bakterium handelt.

5. Die Zusammensetzung zur Verwendung nach einem der Ansprüche 1 bis 4, wobei es sich bei dem Kulturextrakt um einen Kulturüberstand handelt.

6. Die Zusammensetzung zur Verwendung nach Anspruch 5, wobei diese Zusammensetzung ein lebendes Coprococcus-Bakterium und dessen Kulturüberstand, vorzugsweise ein lebendes Coprococcus-Bakterium und dessen Kulturüberstand, umfasst.

7. Die Zusammensetzung zur Verwendung nach einem der Ansprüche 1 bis 6, wobei die Zusammensetzung ferner ein oder mehrere zusätzliche bakterielle Probiotika umfasst, die einen AhR-Agonisten produzieren können und ausgewählt sind aus der Gruppe bestehend aus Bakterien der Genera *Allobaculum, Adlercreutzia, Anaerostipes, Bifidobacterium, Propionibacterium, Bacteroides, Eubacterium, Enterococcus, Ruminococcus* und *Faecalibacterium, Escherichia coli,* und Milchsäurebakterien wie Bakterien der Genera *Lactobacillus* and *Streptococcus,* vorzugsweise ausgewählt aus der Gruppe bestehend aus Bakterien der Genera *Allobaculum, Adlercreutzia, Propionibacterium, Enterococcus, Escherichia coli, Lactobacillus* und *Streptococcus.*

8. Die Zusammensetzung zur Verwendung nach einem der Ansprüche 1 bis 7, wobei die Zusammensetzung ferner ein oder mehrere zusätzliche bakterielle Probiotika umfasst, ausgewählt aus der Gruppe bestehend aus Stämmen, die unter den CNCM-Hinterlegungsnummern CNCM 1-5019, CNCM 1-5020, CNCM 1-5021, CNCM 1-5022 and CNCM 1-5023, *Ruminococcus gnavus* ATCC 29149, *Lactobacillus salivarius* DSM 20555, *Lactobacillus reuteri* DSM 20016, *Lactobacillus gasseri* DSM 20243, *Faecalibacterium prausnitzii* A2-165, *Escherichia coli* MG1665, *Anaerostipes hadrus* DSM 3319, *Anaerostipes caccae* DSM 14662, *Anaerostipes butyraticus* DSM 22094 und *Allobaculum stercoricanis* DSM 13633 verfügbar sind..

9. Die Zusammensetzung zur Verwendung nach einem der Ansprüche 1 bis 6, wobei die Zusammensetzung keine weiteren Bakterien enthält.

10. Die Zusammensetzung zur Verwendung nach einem der Ansprüche 1 bis 9, wobei die Zusammensetzung oral oder rektal verabreicht wird.

11. Die Zusammensetzung zur Verwendung nach einem der Ansprüche 1 bis 10, wobei es sich bei der Erkrankung um metabolisches Syndrom handelt.

12. Zusammensetzung zur Verwendung nach einem der Ansprüche 1 bis 10, wobei es sich bei der Erkrankung um eine mit dem metabolischen Syndrom assoziierte Störung handelt, die ausgewählt ist aus der Gruppe bestehend aus Zusammensetzung zur Verwendung nach einem der Ansprüche 1 bis 10, wobei es sich bei der Erkrankung um eine mit dem metabolischen Syndrom assoziierte Störung handelt, die ausgewählt ist aus der Gruppe bestehend aus Herz-Kreislauf-Erkrankungen, Insulinresistenz, Glukoseintoleranz, nichtalkoholischer Fettlebererkrankung und Lipodystrophie.

13. Die Zusammensetzung zur Verwendung nach einem der Ansprüche 1 bis 10, wobei es sich bei der Erkrankung um eine entzündliche Darmerkrankung handelt, vorzugsweise ausgewählt aus der Gruppe bestehend aus Morbus Crohn, Colitis ulcerosa, unbestimmter Colitis (IC), anderen nichtinfektiösen Gastroenteritiden, Enteritis, Enterokolitis und Colitis sowie Pouchitis

14. Die Zusammensetzung zur Verwendung nach einem der Ansprüche 1 bis 10, wobei es sich bei der Erkrankung um eine entzündliche Darmerkrankung, ausgewählt aus Morbus Crohn und Colitis ulcerosa, handelt.

15. *Coprococcus* comes-Stamm, hinterlegt bei der Deutschen Sammlung von Mikroorganismen und Zellkulturen (DSMZ) unter der Zugangsnummer DSM 33359, oder eine funktionell äquivalente Mutante davon.

## Revendications

1. Une composition comprenant une bactérie *Coprococcus* et/ou un extrait de culture de celle-ci présentant une activité d'agoniste AhR, pour une utilisation dans le traitement d'une maladie choisie dans le groupe constitué du syndrome métabolique et des désordres associés choisis dans le groupe constitué des maladies cardiovasculaires, la résistance à l'insuline, l'intolérance au glucose, la stéatose hépatique non alcoolique, et la lipodystrophie, et des maladies inflammatoires de l'intestin.

2. La composition pour une utilisation selon la revendication 1, dans laquelle la bactérie est une bactérie *Coprococcus* comes.

3. La composition pour une utilisation selon la revendication 2, dans laquelle la bactérie est la souche *Coprococcus comes* déposée au Deutsche Sammlung van Mikroorganismen und Zellkulturen (DSMZ) sous le numéro d'entrée DSM 33359, ou un mutant de celle-ci fonctionnellement équivalent.

4. La composition pour une utilisation selon l'une quelconque des revendications 1 à 3, dans laquelle la bactérie *Coprococcus* est une bactérie Coprococcus vivante.

5. La composition pour une utilisation selon l'une quelconque des revendications 1 à 4, dans laquelle l'extraite de culture est un surnageant de culture.

6. La composition pour une utilisation selon la revendication 5, dans laquelle ladite composition comprend une bactérie Coprococcus vivante et un surnageant de culture de celle-ci, de préférence une bactérie *Coprococcus comes* vivante et un surnageant de culture de celle-ci.

7. La composition pour une utilisation selon l'une quelconque des revendications 1 à 6, dans laquelle la composition comprend en outre un ou plusieurs probiotiques bactériens supplémentaires capables de produire un agoniste d'AhR et choisis dans le groupe constitué des bactéries appartenant aux *genres Allobaculum, Adlercreutzia, Anaerostipes, Bifidobacterium, Propionibacterium, Bacteroides, Eubacterium, Enterococcus, Ruminococcus* et *Faecalibacterium, Escherichia coli,* et les bactéries lactiques telles que les bactéries appartenant aux genres *Lactobacillus* et *Streptococcus,* de préférence choisis dans le groupe constitué des bactéries appartenant aux genres *Allobaculum, Adlercreutzia, Propionibacterium, Enterococcus, Escherichia coli, Lactobacillus* and *Streptococcus.*

8. La composition pour une utilisation selon l'une quelconque des revendications 1 à 7, dans laquelle la composition comprend en outre un ou plusieurs probiotiques bactériens supplémentaires choisis dans le groupe constitué des souches disponibles sous les numéros de dépôt CNCM CNCM 1-5019, CNCM 1-5020, CNCM 1-5021, CNCM 1-5022 and CNCM 1-5023, *Ruminococcus gnavus* ATCC 29149, *Lactobacillus salivarius* DSM 20555, *Lactobacillus reuteri* DSM 20016, *Lactobacillus gasseri* DSM 20243, *Faecalibacterium prausnitzii* A2-165, *Escherichia coli* MG1665, *Anaerostipes hadrus* DSM 3319, *Anaerostipes* caccae DSM 14662, *Anaerostipes butyraticus* DSM 22094 and *Allobaculum stercoricanis* DSM 13633.

9. La composition pour une utilisation selon l'une quelconque des revendications 1 à 6, dans laquelle la composition ne comprend aucune bactérie supplémentaire.

10. La composition pour une utilisation selon l'une quelconque des revendications 1 à 9, dans laquelle ladite composition est pour être administrée par voie orale ou rectale.

11. La composition pour une utilisation selon l'une quelconque des revendications 1 à 10, dans laquelle la maladie est le syndrome métabolique.

12. La composition pour une utilisation selon l'une quelconque des revendications 1 à 10, dans laquelle la maladie est un désordre associé au syndrome métabolique, ledit désordre étant choisi dans le groupe constitué des maladies cardiovasculaires, de la résistance à l'insuline, de l'intolérance au glucose, de la stéatose hépatique non alcoolique et de la lipodystrophie.

13. La composition pour une utilisation selon l'une quelconque des revendications 1 à 10, dans laquelle la maladie est une maladie inflammatoire de l'intestin, de préférence choisie dans le groupe constitué de la maladie de Crohn, la colite ulcéreuse, la colite indéterminée, d'autres gastro-entérites, entérites, entérocolites et colites non infectieuses, et la pochite.

14. La composition pour une utilisation selon l'une quelconque des revendications 1 à 10, dans laquelle la maladie est une maladie inflammatoire de l'intestin choisie parmi la maladie de Crohn et la colite ulcéreuse.

15. Souche de *Coprococcus* comes déposée au Deutsche Sammlung van Mikroorganismen und Zellkulturen (DSMZ) sous le numéro d'entrée DSM 33359, ou un mutant de celle-ci fonctionnellement équivalent.
